# EUROPEAN PATENT SPECIFICATION

(11) **EP 4 384 304 B1**
(45) Date of publication and mention of the grant of the patent: **29.07.2026**
(21) Application number: 22856733.5
(22) Date of filing: 05.08.2022
(51) Int. Cl.: B01D 61/38, A61B 5/15, B01L 3/02, C02F 3/20, G01N 1/28, G01N 1/38

(54) **APPARATUS AND METHOD FOR REMOVING BUBBLES FROM FLUID SAMPLE**
VORRICHTUNG UND VERFAHREN ZUR ENTFERNUNG VON BLASEN AUS EINER FLÜSSIGKEITSPROBE
APPAREIL ET PROCÉDÉ POUR ÉLIMINER DES BULLES D'UN ÉCHANTILLON DE FLUIDE

(30) Priority: 12.08.2021 US 202163232365 P; 16.09.2021 US 202163244987 P
(43) Date of publication of application: 19.06.2024
(73) Proprietor: Siemens Healthcare Diagnostics Inc., Tarrytown, NY 10591 (US)
(72) Inventor: COX, Janine, Stoughton, Massachusetts 02072 (US); DESILVA, Justin, Mendon, Massachusetts 01756 (US)
(74) Representative: Schweitzer, Klaus
(86) International application number: PCT/US2022/074578
(87) International publication number: WO 2023/019086

(56) References cited:
- WO-A1-98/23353
- GB-A- 2 043 478
- US-A- 3 803 810
- US-A- 4 572 210
- US-A1- 2002 127 147

## Description

### CROSS-REFERENCE TO RELATED APPLICATIONS

This application claims benefit under 35 USC § 119(e) of U.S. Provisional Application No. 63/244,987, filed September 16, 2021 and U.S. Provisional Application No. 63/232,365, filed August 12, 2021.

### BACKGROUND

Blood sampling is a common health care procedure typically used in hospital and laboratory settings to determine the physiological and biochemical condition of a patient. Blood sampling is essential to the diagnosis and treatment of patients suspected of a wide variety of disorders. Blood samples are analyzed by fluid testing devices, such as blood analyzers, to detect clinically significant variations in blood components, e.g., plasma, red blood cells, white blood cells, and platelets, or other characteristics, such as blood gas conditions. Analysis of a blood sample for blood gas conditions provides information, such as the amount of oxygen and carbon dioxide in the blood, and may also determine the pH of a blood sample. Imbalances in the oxygen, carbon dioxide, or pH levels of a blood sample may indicate particular pathological conditions or stage of disease progression.

Blood samples are typically collected from a central or arterial line using blood collection syringes having hypodermic needles or vacuum tubes coupled to a needle assembly. However, blood collection syringes are prone to have bubbles of air within the syringe. The bubbles in the barrel and tip of the syringe may alter the blood sample and interfere with the analysis of blood samples. Regarding blood gas analysis, bubbles trapped in a syringe may cause a blood analyzer to produce erroneous results. To obtain accurate results, blood samples need to be mixed thoroughly and all air must be expelled before mixing. Bubbles are typically expelled by tapping the sides of the syringe to force the air or gas to the top of the syringe. Once the bubbles have reached the top of the syringe, a piece of gauze or tissue is put over the top to force the gas (and some blood) out of the syringe. This method poses not only a biohazard risk because the blood sample is freely flowing out of the top of the syringe, but also an exposure risk, as the tip of the syringe is still open to ambient air. When a sample is in contact with ambient air, the sample begins to equilibrate the surrounding gases. Thus, when an improperly de-bubbled sample is tested on a blood gas analyzer, the results may not reflect the actual patient condition.

Further, blood analyzers often may not permit direct sample input via a syringe or vacuum tube. There, the syringe and/or vacuum tube is merely an intermediate container for the blood sample, and at least a portion of the blood sample must be removed and transferred to a secondary sample container (e.g., an adapter) capable of being accommodated by the blood analyzer. Transferring the blood sample to a secondary sample container presents its own exposure risks and may increase the likelihood of obtaining erroneous results.

US 3,803,810 A1 discloses a liquid-gas separator and filter that is capable of separating gases and liquids and of venting the gases in any position of the separator. Gas entering a fluid chamber can leave the chamber through either of liquid-repellent filters while liquid entering the chamber must leave through a liquid wetted filter. Gas passing through the liquid- repellent filters enters chambers and can escape through any of the gas ports. The liquid passing through the liquid-wetted filter passes through another chamber and a passage to a liquid outlet. Such liquid is filtered and entirely-free from gas. The gas is entirely free from liquid by virtue of the separating effect of the liquid-repellent and liquid-wetted filter materials. US 4,572,210 A discloses a syringe device for obtaining a gas-free blood sample. The syringe has a tubular body connected to a hypodermic needle. The syringe comprises plunger means, including a resilient sealing member and a plunger rod. There is a plunger rod passageway extending along a longitudinal axis of the plunger rod and providing a portion of a vent or air flow passage between the interior chamber and the atmosphere. The syringe further comprises a hydrophobic filter covering a sealing member. The physical properties of the hydrophobic filter are such that air is free to pass the filter until such time as the filter is wetted. Once the blood contacts the filter, the wetted portions of the filter are impervious to air and aqueous matter, like blood. Air cannot cross the interface defined by the filter back into the interior chamber once the filter is wetted.

A need exists for an apparatus and method for removing bubbles from a fluid sample. It is to such an apparatus and method that the inventive concepts disclosed and claim herein are directed.

### SUMMARY OF THE INVENTIVE CONCEPTS

The inventive concepts disclosed and claimed herein generally relate to an apparatus according to claim 1 for removing bubbles from a fluid sample having a liquid portion and a gas portion. The apparatus includes a barrel and a filter member. The filter member cooperates with the barrel to provide at least one liquid passage between the filter member and the barrel. The barrel has a first end, a second end, a sidewall, and an inner surface. The sidewall extends between the first end and the second end of the barrel, and the inner surface of the barrel defines an internal chamber. The first end of the barrel has an inlet opening and the second end has an outlet opening. The filter member is disposed within the internal chamber so the filter member defines an inlet side and an outlet side of the internal chamber. The filter member is positioned between the first end and the second end of the filter member. The filter member has at least one gas-permeable, liquid-impermeable membrane to permit at least a portion of the gas portion of the fluid sample to pass across the filter member from the inlet side to the outlet side of the internal chamber. The filter member is slidably disposed in the internal chamber of the barrel so as to be movable from a first position to a second position. In the first position, the filter member cooperates with the barrel to provide a fluid-tight seal across the filter member preventing the liquid portion of the fluid sample from passing from the inlet side to the outlet side as the fluid sample is passed into the internal chamber via the inlet opening to separate at least a portion of the gas portion from the liquid portion of the fluid sample. In the second position, the filter member cooperates with the barrel to provide at least one liquid passage between the filter member and the barrel to allow the liquid portion of the fluid sample to pass from the inlet side to the outlet side. The liquid passage is defined by a body and a plurality of grooves extending along at least a portion of the inner surface of the barrel. The barrel further includes a second gas-permeable, liquid-impermeable membrane that is secured to the barrel adjacent the second end of the barrel. The second gas-permeable, liquid-impermeable membrane provides a fluid-tight seal across the outlet opening to prevent the liquid portion of the fluid sample from passing into the outlet opening from the internal chamber as the liquid portion of the fluid sample is passed from the inlet side to the outlet side via the liquid passage. The second gas-permeable, liquid-impermeable membrane is pierceable by a sample probe so the probe may be passed through the gas-permeable, liquid-impermeable membrane to the outlet side to withdraw the liquid portion of the fluid sample from the outlet side of the internal chamber.

### BRIEF DESCRIPTION OF THE DRAWINGS

To assist those of ordinary skill in the relevant art in making and using the inventive concepts disclosed herein, reference is made to the appended drawings and schematics, which are not intended to be drawn to scale, and in which like reference numerals are intended to refer to the same or similar elements for consistency. For purposes of clarity, not every component may be labeled in every drawing. Certain features and certain views of the figures may be shown exaggerated and not to scale or in schematic in the interest of clarity and conciseness. In the drawings:
FIG. 1 is a side perspective view of an exemplary embodiment of an apparatus for removing bubbles according to the inventive concepts disclosed herein shown coupled to a sample receiving assembly.
FIG. 2A is a longitudinal, cross-sectional view of the apparatus of FIG. 1 coupled to a collection syringe illustrating positioning of a filter member and a plunger assembly prior to removal of bubbles from the fluid sample.
FIG. 2B is a longitudinal, cross-sectional view of the apparatus of FIG. 1 coupled to a collection syringe illustrating positioning of the filter member and plunger assembly following removal of bubbles from the fluid sample.
FIG. 2C is a longitudinal, cross-sectional view of the apparatus of FIG. 1 coupled to a collection syringe illustrating the insertion of a probe into the apparatus following removal of bubbles from of the fluid sample.
FIG. 3A is a longitudinal, cross-sectional view of the apparatus of FIG. 1 illustrating a position of the filter member prior to removal of bubbles from the fluid sample.
FIG. 3B is a is a longitudinal, cross-sectional view of the apparatus of FIG. 1 illustrating a position of the filter member following removal of bubbles from the fluid sample but prior to the insertion of a probe into the apparatus.
FIG. 3C is a longitudinal, cross-sectional view of the apparatus of FIG. 1 illustrating the insertion of the probe into the apparatus following removal of bubbles from the fluid sample.
FIG. 4 is a perspective view of the apparatus.
FIG. 5A is an exploded, cross-sectional view of the apparatus.
FIG. 5B is a cross-sectional view taken along line 5B-5B of FIG. 3C.
FIG. 6A is a perspective view of an exemplary embodiment of the filter member according to the inventive concepts disclosed herein.
FIG. 6B is a cross-sectional view taken along line 6B-6B of FIG. 6A.
FIG. 7 is a perspective view of another embodiment of a filter member according to the inventive concepts disclosed herein.
FIG. 8A is a perspective view of another embodiment of a filter member according to the inventive concepts disclosed herein.
FIG. 8B is a cross-sectional view taken along line 8B-8B of FIG. 8A

### DETAILED DESCRIPTION OF EXEMPLARY EMBODIMENTS

Unless otherwise defined, scientific and technical terms used in connection with the presently disclosed and claimed inventive concept(s) shall have the meanings commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses and chemical analyses.

All the articles, compositions and/or methods disclosed and claimed herein can be made and executed without undue experimentation, given the present disclosure.

As utilized under the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the inherent variation of error for the device, the method being employed to determine the value, or the variation that exists among the study subjects.

The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

As used herein, the term "sample" and variations thereof is intended to include biological tissues, biological fluids, chemical fluids, chemical substances, suspensions, solutions, slurries, mixtures, agglomerations, tinctures, slides, powders, or other preparations of biological tissues or fluids, synthetic analogs to biological tissues or fluids, bacterial cells (prokaryotic or eukaryotic), viruses, single-celled organisms, lysed biological cells, fixed biological cells, fixed biological tissues, cell cultures, tissue cultures, genetically engineered cells and tissues, genetically engineered organisms, and combinations thereof, for example.

In the following detailed description of embodiments of the inventive concept, numerous specific details are set forth in order to provide a more thorough understanding of the inventive concept. However, it will be apparent to one of ordinary skill in the art that the inventive concept within the disclosure may be practiced without these specific details. In other instances, well-known features have not been described in detail to avoid unnecessarily complicating the instant disclosure.

Finally, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Described herein, and shown in the accompanying figures, are several embodiments of the apparatus of the presently claimed and disclosed inventive concepts which may be used in association with collection syringes and fluid sample analyzers for removing bubbles of air or other gases from a fluid sample for analysis by a fluid sample analyzer. The fluid sample is generally from a biological source. A "fluid" refers to any substance that has no fixed shape and yields easily to external pressure.

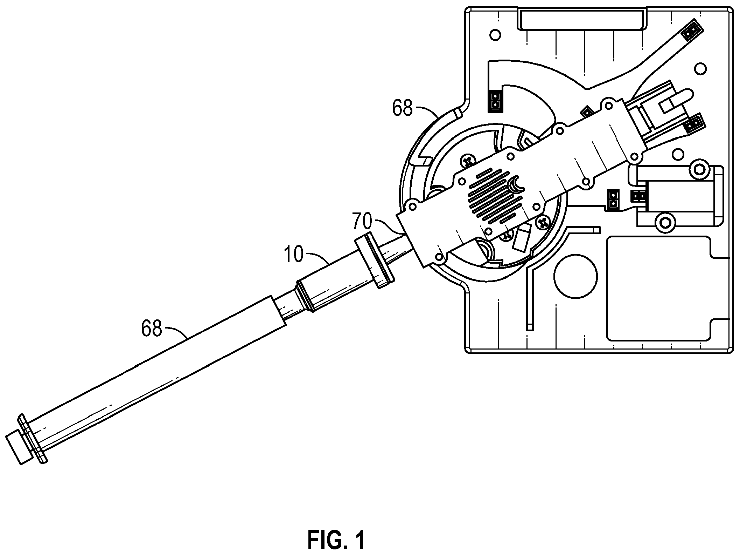

Referring now to the drawings, and more particularly to FIGS. 1-5, shown therein is an exemplary embodiment of an apparatus 10 for removing bubbles from a fluid sample constructed in accordance with the inventive concepts disclosed and claimed herein. The apparatus 10 includes a barrel 12, a nozzle cap 14, and a filter member 16.

The barrel 12 includes a first end 18, a second end 20, a sidewall 22, and an inner surface 24. The barrel 12 may be of any suitable size and shape, and formed of any suitable material, such as, without limitation, plastics such as polycarbonate, polystyrene, polyacrylates, and polyurethane, or medical-grade polymers. The sidewall 22 of the barrel 12 extends between the first end 18 and the second end 20 of the barrel 12. The inner surface 24 of the barrel 12 defines an internal chamber 26. The first end 18 has an inlet opening 28 and the second end 20 has an outlet opening 30. The internal chamber 26 may be of any suitable size and shape to contain a fluid sample 32. The fluid sample 32 may be, for example, blood, serum, plasma, or other bodily fluids. The fluid sample 32 contains a gas portion and a liquid portion. The gas portion of the fluid sample 32 may be, for example, air or other gases. A portion of the gas portion may form bubbles in the fluid sample

The inlet opening 28 and the outlet opening 30 may have a cross-section of any suitable geometry, including, but not limited to, circular, oval, square, or rectangular. The inlet opening 28 and the outlet opening 30 may be molded or cut into the barrel 12, or otherwise pre-fabricated. The inlet opening 28 may be formed to prevent coagulation as the fluid sample 32 is passed into the internal chamber 26 via the inlet opening 28.

The outlet opening 30 may be provided with a nozzle cap 14. The nozzle cap 14 includes an annular wall 36 and a tubular portion 38 having a bore 40 extending therethrough. The bore 40 may have a cross-section of any suitable geometry, including, but not limited to, circular, oval, square, or rectangular. The bore 40 may be sized to have a diameter adapted to slidably axially receive a sample probe. The base of the tubular portion 38 flares outwardly and merges with the annular wall 36 at a rim 42, the annular wall 36 tapers downwardly to form an inverted frusto-conical section. The nozzle cap 14 may be releasably coupled to the outlet opening 30 such that the bore 40 is aligned with the outlet opening 30 to permit fluid communication with the internal chamber 26.

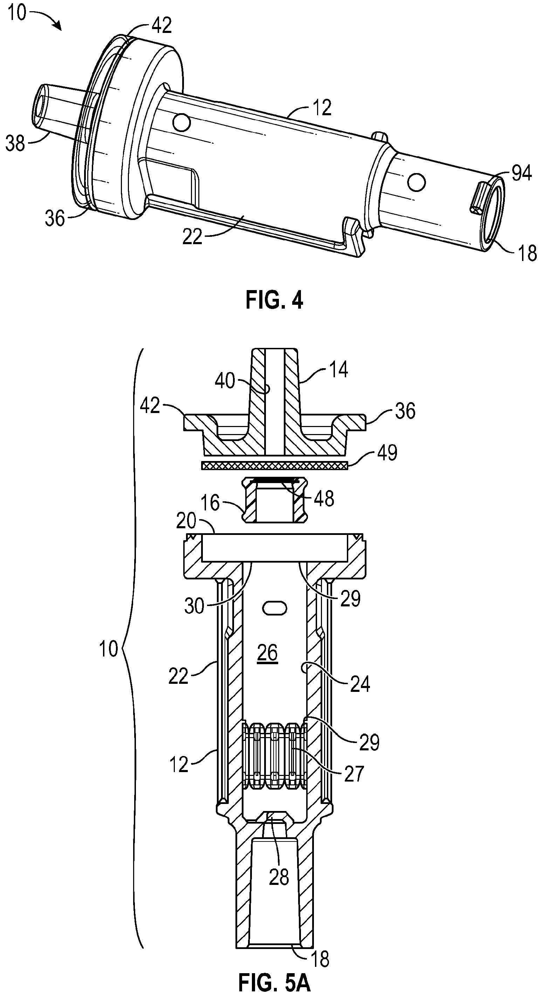

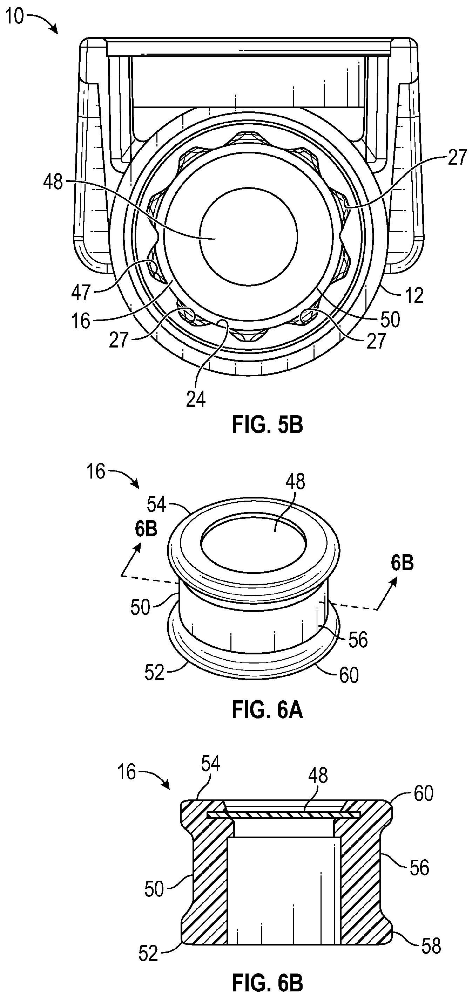

Referring now to FIGS. 5A and 5B, shown therein is an exploded, cross-sectional view of the apparatus 10 and a cross-sectional view of the barrel 12, respectively. The inner surface 24 of the barrel 12 includes a plurality of grooves 27 and a stop member 29. The grooves 27 extend circumferentially about the inner surface 24 of the barrel 12 and extend longitudinally along at least a portion of the inner surface 24 from the first end 18 towards the second end 20 of the barrel 12. The grooves 27 are spaced apart in a parallel relationship to each other.

As will be discussed below, the groves 27 cooperate with the filter member 16 to define at least one liquid passage between the filter member 16 and the barrel 12 to allow the liquid portion of the fluid sample to pass from the inlet side 44 of the internal chamber 26 to the outlet side 46 thereof. It will be appreciated that the number and size of the grooves 27 may be varied. The grooves 27 may be molded or machined into the inner surface 24, or otherwise pre-fabricated. The grooves 27 terminates at or near the stop member 29. The stop member 29 is positioned intermediate the first end 18 and the second end 20 of the barrel 12. The stop member 29 extends radially from the inner surface 24 into the internal chamber 26. The stop member 29 includes a surface to engage at least one surface of the filter member 16 and prevents the movement of the filter member 16 beyond the stop member 29 and prior to contact of the filter member 16 with the second end 20 of the barrel 12.

The filter member 16 is disposed within the internal chamber 26 so the filter member 16 defines an inlet side 44 and an outlet side 46 of the internal chamber 26. The filter member 16 is positionable between the first end 18 and the second end 20 of the barrel 12. The filter member 16 includes at least one gas-permeable, liquid-impermeable membrane 48.

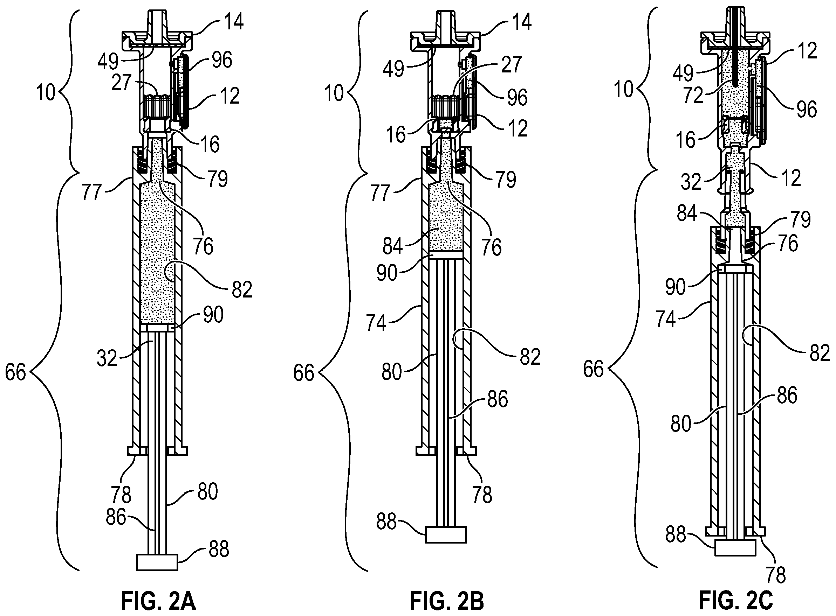

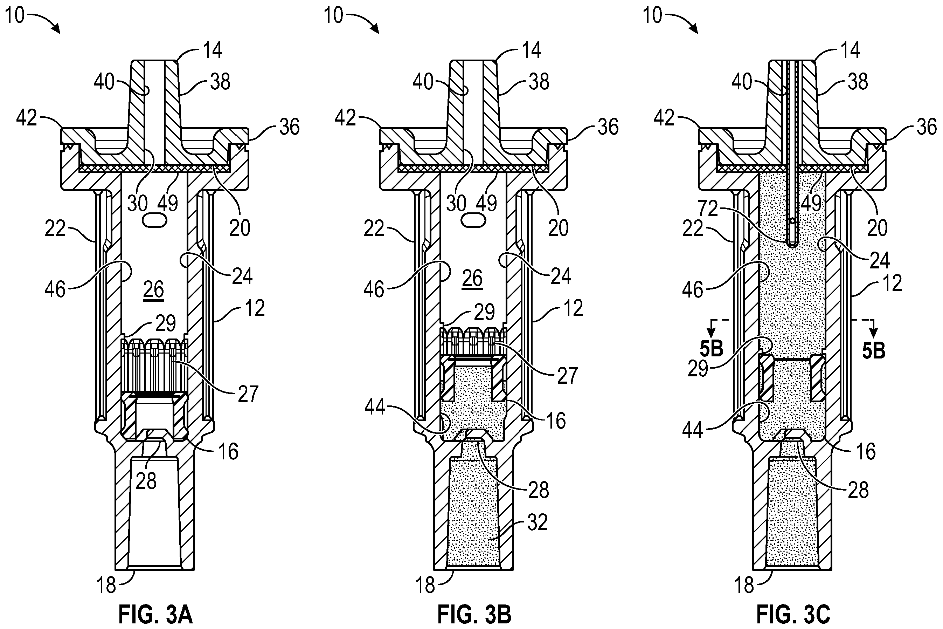

The filter member 16 is slidable from a first position (FIGS. 2A and 3A) to a second position (FIGS. 2C and 3C). In the first position, the filter member 16 cooperates with the barrel 12 to provide a fluid-tight seal across the filter member 16 to prevent the liquid portion of the fluid sample 32 from passing from the inlet side 44 to the outlet side 46 of the internal chamber 26 as the fluid sample 32 is passed into the internal chamber 26 via the inlet opening 28 to separate at least a portion of the gas portion from the liquid portion of the fluid sample 32. In the second position, the filter member 16 cooperates with the barrel 12 to provide at least one liquid passage 47 (FIG. 5B) between the filter member 16 and the barrel 12 to allow the liquid portion of the fluid sample 32 to pass from the inlet side 44 to the outlet side 46 of the internal chamber 26. In the embodiment illustrated in FIG. 5B, the filter member 16 cooperates with the barrel 12 to provide a plurality of liquid passages 47 (FIG. 5B) between the filter member 16 and the barrel 12 to allow the liquid portion of the fluid sample 32 to pass from the inlet side 44 to the outlet side 46 of the internal chamber 26.

Referring now to FIGS. 6A and 6B, shown therein is a perspective and cross-sectional view, respectively, of an exemplary embodiment of the filter member 16. The filter member 16 comprises a body 50 having a first end 52, a second end 54, and a sidewall 56 extending from the first end 52 to the second end 54. The sidewall 56 of the body 50 defines a passageway 58 extending through the body 50 from the first end 52 to the second end 54. The sidewall 56 of the body 50 has at least two annular projections 60 extending radially outwardly in slidable, sealing contact with the inner surface 24 of the barrel 12. As shown in FIGS. 6A and 6B, the body 50 may have two annular projections 60 spaced apart from each other. The annular projections 60 may include convex or concave features.

The body 50 of the filter member 16 may be formed of any suitable material, such as, without limitation, a rubber, an elastomer, a polyolefin-based resin, a fluorine-based resin, or a polyester-based resin. The elastomer may include, for example, a polyvinyl chloride-based elastomer, a polyolefin-based elastomer, a styrene-based elastomer, a polyester-based elastomer, a polyamide-based elastomer, a polyurethane-based elastomer, or a mixture thereof.

The filter member 16 further includes a gas-permeable, liquid-impermeable membrane 48 which extends across the entirety of the passageway 58. In one embodiment, the gas-permeable, liquid-impermeable membrane 48 is secured to the body 50 adjacent the second end 54 of the body 50, as shown in FIG. 6B.

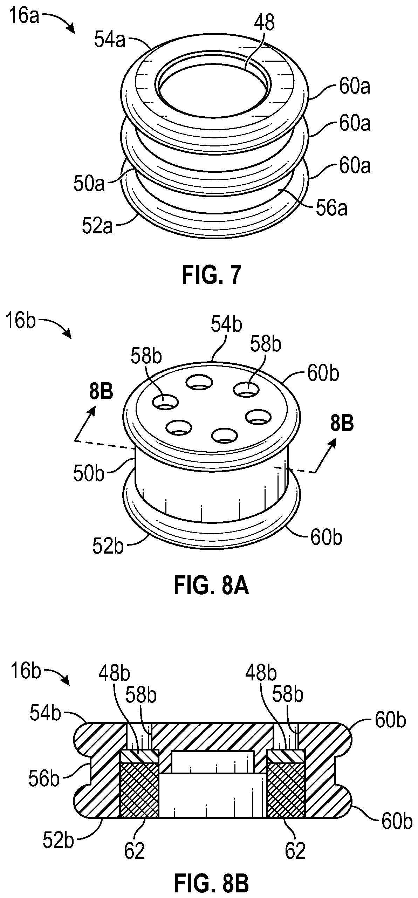

Referring now to FIG. 7, shown therein is a perspective view of another embodiment of a filter member 16a constructed in accordance with the inventive concepts disclosed and claimed herein. Similar to previously described embodiments, the filter member 16a comprises a body 50a having a first end 52a, a second end 54a, and a sidewall 56a extending from the first end 52a to the second end 54a. The sidewall 56a of the body 50a defines a passageway (not shown) extending through the body 50a from the first end 52a to the second end 54a. As in FIGS. 6A and 6B of the previously described embodiment, the body 50a has at least two annular projections 60a extending radially outwardly in slidable, sealing contact with the inner surface 24 of the barrel 12. As shown in FIG. 7, the body 50a may have three annular projections 60a spaced apart from each other. The filter member 16a further includes the gas-permeable, liquid-impermeable membrane 48 which extends across the entirety of the passageway. In this embodiment, the gas-permeable, liquid-impermeable membrane 48 is secured to the body 50a adjacent the second end 54a of the body.

Referring now to FIGS. 8A and 8B, shown therein is a perspective and cross-sectional view, respectively, of another embodiment of a filter member 16b. The filter member 16b comprises a body 50b having a first end 52b, a second end 54b, and a sidewall 56b extending from the first end 52b to the second end 54b. Similar to previously described embodiments, the body 50b has at least two annular projections 60b extending radially outwardly in slidable, sealing contact with the inner surface 24 of the barrel 12. The sidewall 56b of the body 50b defines a plurality of passageways 58b extending through the body 50b from the first end 52b to the second end 54b. The plurality of passageways 58b may be in a parallel relationship to one another, as shown in FIG. 8B. Further, the filter member 16b may include a plurality of gas-permeable, liquid-impermeable membranes 48b with at least one of the gas-permeable, liquid-impermeable membranes 48b extending across each of the plurality of passageways 58b of the body 50b. The filter member 16b may further include a plurality of porous filter material 62 positioned between the first end 52b of the body 50b and each of the plurality of gas-permeable, liquid-impermeable membranes 48b to prevent solid particulate from contacting the plurality of gas-permeable, liquid-impermeable membranes 48b, as shown in FIG. 8B.

A gas-permeable, liquid impermeable membrane 49 is secured to the barrel 12 adjacent the second end 20 of the barrel 12 to provide a fluid-tight seal across the outlet opening 30 to prevent the liquid portion of the fluid sample 32 from passing into the outlet opening 30 from the internal chamber 26 as the liquid portion of the fluid sample 32 is passed from the inlet side 44 to the outlet side 46 via the liquid passage 47. The gas-permeable, liquid impermeable membrane 49 is pierceable so a sample probe 72 may be passed through the gas-permeable, liquid impermeable membrane 49 from the outlet side 46 of the internal chamber 26 to withdraw the liquid portion of the fluid sample 32 from the outlet side 46 of the internal chamber 26.

The gas-permeable, liquid-impermeable membranes 48 and 49 may be formed of any suitable material, such as, without limitation, polytetrafluoroethylene, polyvinylidene fluoride, polyvinylchloride, polyolefins like polypropylene, polyethylene, polymethylpentene, polyamides, polysulfones, polyetheretherketones, polycarbonates, and combinations including any of these. In one embodiment, the gas-permeable, liquid-impermeable membranes 48 and 49 are formed from a material comprising at least one of polytetrafluorethylene, polypropylene, and polyethylene. The gas-permeable, liquid-impermeable membrane 49 may have a thickness suitable for allowing puncture upon application of a mechanical force. The gas-permeable, liquid-impermeable membrane 48 permits at least a portion of the gas portion of the fluid sample 32, which forms bubbles, to pass across the filter member 16 from the inlet side 44 to the outlet side 46 of the internal chamber 26. The gas-permeable, liquid-impermeable membrane 48 also provides a fluid-tight seal across the filter member 16 to prevent the liquid portion of the fluid sample 32 from passing from inlet side 44 to the outlet side 46 as the fluid sample 32 is passed into the internal chamber 26 via the inlet opening 28 to separate at least a portion of the gas portion from the liquid portion of the fluid sample 32

As shown in FIG. 1, the apparatus 10 may be used in association with a collection syringe 66 and a fluid sample analyzer 68 to remove bubbles from the fluid sample 32 having a liquid portion and a gas portion. Although, FIG. 1 shows the apparatus 10 associated with the collection syringe 66 and the fluid sample analyzer 68, those skilled in the art will understand and appreciate that the apparatus 10 may independently be associated with collection devices other than the collection syringe 66, such as, for example, a vacuum tube, and medical devices other than the fluid sample analyzer 68. The fluid sample analyzer 68 may be any suitable fluid testing device, such as microfluidic devices, blood gas analyzers, hematology analyzers, urine chemistry analyzers, and the like.

The fluid sample analyzer 68 includes a sample input port 70 and a sample probe 72. The sample probe 72 may be axially slidable relative to the sample input port 70. The sample input port 70 may be sized to receive and detachably secure at least a portion of the nozzle cap 14, as shown in FIG. 1. The sample probe 72 may be extended to pass through the gas-permeable, liquid impermeable membrane 49 secured adjacent the second end 20 of the barrel 12 to the outlet side 46 to withdraw at least a portion of the fluid sample 32 into the fluid sample analyzer 68 from the outlet side 46 of the internal chamber 26, as shown in FIGS. 2C and 3C. The sample probe 72 may be of a length compatible with sampling from the outlet side 46 of the internal chamber 26.

The collection syringe 66 includes a syringe body 74 having a front end 76, a rear end 78, and a plunger 80. The syringe body 74 defines a reservoir 82 within which the fluid sample 32 may be contained and later expelled via a dispensing opening 84 positioned at the front end 76 of the syringe body 74. The rear end 78 of the syringe body 74 may be open and provided with a body flange 85 to facilitate the collection and expulsion of the fluid sample 32. The syringe body 74 may be any suitable size or shape for collection of fluid samples, such as, for example, a cylindrical shape. The syringe body 74 may also include a collar 77 formed concentrically with the dispensing opening 84 into a cylindrical shape to surround the dispending opening 84. The collar 77 may include an inner peripheral surface in which a threaded engagement portion 79 is formed for engaging the apparatus 10. The syringe body 74 may be constructed of any suitable material, such as glass or plastic. The syringe body 74 may have an outer diameter adapted to coaxially slide within inlet opening 28 of the apparatus 10.

The plunger 80 may include a shaft 86 that terminates at one end in a plunger flange 88 to facilitate the collection and expulsion of the fluid sample 32. The shaft 86 may, for example, have a cylindrical shape or a columnar shape, and may have a cross-section of a polygonal shape, such as a square, pentagonal, hexagonal, or cruciform shape. The plunger 80 may further include a plunger seal 90 secured to the shaft 86 opposite the plunger flange 88. The plunger 80 may be removably disposed within the syringe body 74 and may be selectively movable within the reservoir 82. The plunger seal 90 has a diameter that permits the plunger seal 90 to create a fluid-tight seal when positioned within the reservoir 82 such that the liquid sample 32 may not move past the plunger seal 90. Further, the plunger seal 90 prevents ambient air from moving from the rear end 78 of the syringe body 74 in a direction past the plunger seal 90. The plunger 80 may be axially displaced relative to the syringe body 74. Movement of the plunger 80 from the rear end 78 to the front end 76 of the syringe body 74 may cause at least a portion of the fluid sample 32 to be expelled from the reservoir 82 and introduced into the inlet opening 28 of the apparatus 10 via the dispensing opening 84. The plunger 80 may be constructed of any suitable polymeric material known in the art.

To remove the gas portion (i.e., bubbles) of the fluid sample 32 from the liquid portion, the collection syringe 66, containing a volume of the fluid sample 32 within the reservoir 82, is releasably attached to the inlet opening 28 of the barrel 12, as shown in FIG. 2A. As shown in FIGS. 2A-2C, the front end 76 of the syringe body 74 may be releasably connected to the apparatus 10 by way of the threaded engagement portion 79. As shown in FIG. 4, the barrel 12 may include a barrel connection portion 94. In one embodiment, the threaded engagement portion 79 of the syringe body 74 is a male luer connection and the barrel connection portion 94 is a female luer connector, although other suitable connectors may be utilized. As shown in FIGS. 2A-2C, the threaded engagement portion 79 may detachably engage the barrel connection portion 94 such that significant relative movement between the collection syringe 66 and the apparatus 10 is prevented, and fluid communication between the reservoir 82 and the inlet opening 28 of the apparatus 10 is permitted.

The collection syringe 66 and the apparatus 10 are positioned in an upright orientation with the apparatus 10 above the collection syringe 66 and the bubbles in the fluid sample rise to the top of the fluid sample 32. The plunger 80 of the collection syringe 66 is displaced axially along the reservoir 82 a distance from the rear end 78 towards the front end 76 of the syringe body 74, as shown in FIG. 2A. Movement of the plunger 80 within the reservoir 82 causes at least a portion of the gas portion (i.e., bubbles) of the fluid sample 32 to be expelled from the reservoir 82 and into the internal chamber 26 of the apparatus 10 via the inlet opening 28, passing through the filter member 16. The filter member 16 in the first position cooperates with the barrel 12 to provide a fluid-tight seal across the filter member 16 to prevent the liquid portion of the fluid sample 32 from passing from the inlet side 44 to the outlet side 46 of the internal chamber 26. The gas portion of the fluid sample 32 passes through the filter member 16, and is then ultimately expelled from the internal chamber 26 of the apparatus 10. After a portion of the gas portion of the fluid sample 32 has been displaced from the reservoir 82, the plunger 80 experiences an initial resistive force.

Upon application of sufficient force to overcome the initial resistive force, the plunger 80 is advanced further into the reservoir 82 towards the front end 76 of the syringe body 74, as shown in FIG. 2B, thereby increasing the internal pressure of the reservoir 82. As the internal pressure of the reservoir 82 increases, it produces a force sufficient to cause at least a portion of the liquid portion of the fluid sample 32 to be expelled from the reservoir 82 and into the internal chamber 26 of the barrel 12 via the inlet opening 28. The fluid sample 32 entering the internal chamber 26 may cause the filter member 16 to be displaced axially along the internal chamber 26 towards the second end 20 of the barrel 12, as shown in FIGS. 2B and 3B.

The filter member 16 continues to migrate along the length of the internal chamber 26 before contacting the stop member 29 of the barrel 12 and coming to rest in the second position, as shown in FIGS. 2C and 3C. Once the filter member 16 contacts the stop member 29, the filter member 16 is prevented from moving past the stop member 29. In the second position, the filter member 16 cooperates with the barrel 12 such that the body 50 of the filter member and the grooves 27 extending along the inner surface 24 of the barrel 12 define the liquid passages 47 that permit fluid communication between the inlet side 44 and the outlet side 46 of the internal chamber 26.

In this position, as the fluid sample 32 enters the inlet side 44 of the internal chamber, the liquid portion of the fluid sample 32 infiltrates into the fluid passage 47 and passes between the filter member 16 and the barrel 12 from the inlet side 44 of the internal chamber 26 to the outlet side 46 of the internal chamber 26. The gas-permeable, liquid-impermeable membrane 49, secured to the barrel 12 adjacent the second end 20, prevents the liquid portion of the fluid sample 32 from being discharged from the internal chamber 26.

This arrangement prevents ambient air from entering the internal chamber 26 and prevents the fluid sample 32 from exiting the internal chamber 26 via the outlet opening 30. In some embodiments, the plunger 80 may be partially extended into the reservoir 82 so less than all of the fluid sample 32 is transferred from the reservoir 82 into the internal chamber 26.

Once the liquid portion of the fluid sample 32 has been expelled from the reservoir 82 and is contained within the internal chamber 26 of the apparatus 10, the sample probe 72 of the fluid sample analyzer 68 may be extended from the sample input port 70 and passed through the gas-permeable, liquid impermeable membrane 49 secured adjacent the second end 20 of the barrel 12 to withdraw the liquid portion of the fluid sample 32 from the outlet side 46 of the internal chamber 26, as shown in FIGS. 2C and 3C. In one embodiment, the sample probe 72 pierces the gas-permeable, liquid-impermeable membrane 49 of the barrel 12 to gain fluid access to the outlet side 46 of the internal chamber 26.

In some embodiments, the barrel 12 may further include a lateral flow strip 96, as shown in FIGS. 2A-2C. In those embodiments, the lateral flow strip 96 may be in fluid contact with the internal chamber 26 of the barrel 12 such that the liquid portion of the fluid sample 32 may contact a surface of the lateral flow strip 96. The lateral flow strip 96 may detect, for example, the presence of hemolysis, or produce a color change depending on the time of introduction of the liquid portion of the fluid sample 32 or upon contact with a certain analyte (e.g., plasma). Additional aspects related to the lateral flow strip 96 are disclosed in Ledden et al., "Detection of Hemolysis Using a Chromatographic Detection Pad," U.S. Publication No. 2017/0108516 and in Cox et al., "Detection of Hemolysis Using a Chromatographic Assay Device," U.S. Provisional Application No. 63/366,558.

From the above description, it is clear that the inventive concept(s) disclosed herein is well adapted to carry out the objects and to attain the advantages mentioned herein as well as those inherent in the inventive concept disclosed herein.

## Claims

1. An apparatus (10) for removing bubbles from a fluid sample (32) having a liquid portion and a gas portion, comprising:
a barrel (12) having a first end (18), a second end (20), a sidewall (22) extending between the first end and the second end, and an inner surface (24) defining an internal chamber (26), the first end having an inlet opening (28) and the second end having an outlet opening (30);
a filter member (16) disposed within the internal chamber (26) between the first end and the second end so the filter member (16) defines an inlet side (44) and an outlet side (46) of the internal chamber, the filter member (16) having at least one first gas-permeable, liquid-impermeable membrane (48), the filter member (16) slidable in the internal chamber (26) of the barrel (12) from a first position allowing the gas portion of the fluid sample (32) to pass through the at least one gas-permeable, liquid-impermeable membrane (48) and to a second position allowing the liquid portion of the fluid sample (32) to pass from the inlet side (44) to the outlet side (46); and
a second gas-permeable, liquid-impermeable membrane (49) secured to the barrel adjacent the second end of the barrel (12), the second gas-permeable, liquid-impermeable membrane (49) pierceable by a sample probe (72) so the sample probe (72) may be passed through the second gas-permeable, liquid-impermeable membrane to the outlet side (46) of the internal chamber (26),
wherein the filter member (16) further comprises the body (50) having a first end (52), a second end (54), and a sidewall (56) extending from the first end to the second end, the sidewall (22) of the body (50) defining a passageway (58) extending through the body (50) from the first end to the second end, and wherein the gas-permeable, liquid-impermeable membrane (48) extends across an entirety of the passageway (58), and wherein the sidewall (56) of the body (50) has at least two annular projections (60) extending radially outwardly in slidable contact with the inner surface of the barrel (12), and wherein at least one of the projections (60) is in sealing contact with the inner surface of the barrel (12) when the filter member (16) is in the first position, and wherein the filter member (16) cooperates with the barrel (12) to provide at least one liquid passage (47) between the filter member (16) and the barrel, wherein the liquid passage (47) is defined by a body (50) and a plurality of grooves (27) extending along at least a portion of the inner surface of the barrel (12).

2. An apparatus for removing bubbles from a fluid sample (32) having a liquid portion and a gas portion according to claim 1 in combination with a liquid sample analyzer having the sample probe (72), wherein the second end of the barrel (12) being connected to the liquid sample analyzer.

3. The apparatus of claim 1, wherein the grooves (27) extend circumferentially about the inner surface of the barrel (12), or
wherein the grooves (27) are parallel to one another.

4. The apparatus of claim 1 or 2, wherein the barrel (12) has a stop member to engage the filter member (16) in the second position.

5. The apparatus of claim 1, wherein the gas-permeable, liquid-impermeable membrane (48) is secured to the body (50) adjacent the second end of the body (50).

6. The apparatus of claim 1 or 2, wherein the gas-permeable, liquid-impermeable membrane (48) is formed from a material comprising at least one of polytetraflyorethylene, polypropylene, and polyethylene.

7. The apparatus of claim 1, wherein the body (50) has a plurality of passageways (58) extending through the body from the first end to the second end, and wherein the filter member (16) further has a plurality of gas-permeable, liquid impermeable membranes with at least one of the gas-permeable, liquid impermeable membranes extending across each of the passageways (58) of the body (50).

8. The apparatus of claim 7, wherein the filter member further comprises a plurality of porous filter material positioned between the first end of the body (50) and each of the gas-permeable, liquid impermeable membranes to prevent solid particulate from contacting the gas-permeable, liquid impermeable membranes.

9. The apparatus of claim 7, wherein the plurality of gas-permeable, liquid-impermeable membranes are secured to the body (50) adjacent the second end of the body (50), and/or
wherein the passageways (58) are in a parallel relationship to one another, and/or
wherein the gas-permeable, liquid-impermeable membrane (48) is formed from a material comprising at least one of polytetraflyorethylene, polypropylene, and polyethylene.

10. The apparatus of claim 1 or 2, wherein the second gas-permeable, liquid-impermeable membrane (49) is formed from a material comprising at least one of polytetraflyorethylene, polypropylene, and polyethylene.

11. A method of removing bubbles from a fluid sample (32) having a liquid portion and a gas portion, the method comprising:
receiving a flow of the fluid sample (32) from an inlet opening (28) of a barrel having a first end, a second end, a sidewall (22) extending between the first end and the second end, and an inner surface defining an internal chamber, the first end having the inlet opening (28) and the second end having an outlet opening (30);
passing the fluid sample (32) into the internal chamber (26) via the inlet opening (28);
causing the fluid sample (32) to contact a filter member disposed within the internal chamber, the filter member defining an inlet side (44) and an outlet side (46) of the internal chamber, the filter member having a gas-permeable, liquid-impermeable membrane, the filter member being movable from a first position to a second position;
separating at least a portion of the gas portion of the liquid sample from the liquid portion of the fluid sample (32) by causing the fluid sample to contact the gas-permeable, liquid-impermeable membrane (48) of the filter member in the first position so that at least a portion of the gas portion of the fluid sample (32) passes across the filter member from the inlet side (44) to the outlet side (46) of the internal chamber (26) prior to causing the filter member to be moved to the second position so the liquid portion of the fluid sample (32) passes from the inlet side (44) to the outlet side (46); and
collecting at least a portion of the liquid portion of the fluid sample (32) from the outlet side of the internal chamber, wherein at least a portion of the liquid portion of the fluid sample (32) is collected from the outlet side (46) of the internal chamber (26) by piercing a second gas-permeable, liquid-impermeable membrane (49) secured to the barrel (12) adjacent the second end of the barrel (12) so a sample probe may be passed through the second gas-permeable, liquid-impermeable membrane (49) to the outlet side to withdraw the liquid portion of the fluid sample (32) from the outlet side (46) of the internal chamber,
wherein the filter member (16) further comprises the body (50) having a first end (52), a second end (54), and a sidewall (56) extending from the first end to the second end, the sidewall (22) of the body (50) defining a passageway (58) extending through the body (50) from the first end to the second end, and wherein the gas-permeable, liquid-impermeable membrane (48) extends across an entirety of the passageway (58), and wherein the sidewall (56) of the body (50) has at least two annular projections (60) extending radially outwardly in slidable contact with the inner surface of the barrel (12), and wherein at least one of the projections (60) is in sealing contact with the inner surface of the barrel (12) when the filter member (16) is in the first position, and wherein the filter member (16) cooperates with the barrel (12) to provide at least one liquid passage (47) between the filter member (16) and the barrel,
wherein the liquid passage (47) is defined by a body (50) and a plurality of grooves (27) extending along at least a portion of the inner surface of the barrel (12).

12. The method of claim 11, wherein the filter member further comprises a filter element interposed between the gas-permeable, liquid-impermeable membrane (48) and the inlet side (44) of the internal chamber, the at least one filter element having an inlet port and an outlet port, the inlet port in fluid communication with the inlet side (44) of the internal chamber (26), the outlet port in fluid communication with the gas-permeable, liquid-impermeable membrane to permit at least a portion of the fluid sample (32) to pass across the at least one filter element and to the gas-permeable, liquid-impermeable membrane (48), wherein the gas-permeable, liquid-impermeable membrane preferably is a plurality of gas-permeable, liquid-impermeable membranes (48).

## Patentansprüche

1. Vorrichtung (10) zum Entfernen von Blasen aus einer Fluidprobe (32) mit einem Flüssigkeitsanteil und einem Gasanteil, Folgendes umfassend:
einen Zylinder (12) mit einem ersten Ende (18), einem zweiten Ende (20), einer Seitenwand (22), die sich zwischen dem ersten Ende und dem zweiten Ende erstreckt, und einer Innenfläche (24), die eine Innenkammer (26) definiert, wobei das erste Ende eine Einlassöffnung (28) aufweist und das zweite Ende eine Auslassöffnung (30) aufweist;
ein Filterelement (16), das innerhalb der Innenkammer (26) zwischen dem ersten Ende und dem zweiten Ende angeordnet ist, so dass das Filterelement (16) eine Einlassseite (44) und eine Auslassseite (46) der Innenkammer definiert, wobei das Filterelement (16) mindestens eine erste gasdurchlässige, flüssigkeitsundurchlässige Membran (48) aufweist, wobei das Filterelement (16) in der Innenkammer (26) des Zylinders (12) von einer ersten Position, die es dem Gasanteil der Fluidprobe (32) ermöglicht, durch die mindestens eine gasdurchlässige, flüssigkeitsundurchlässige Membran (48) zu verlaufen, in eine zweite Position, die es dem Flüssigkeitsanteil der Fluidprobe (32) ermöglicht, von der Einlassseite (44) zur Auslassseite (46) zu verlaufen, verschiebbar ist; und
eine zweite gasdurchlässige, flüssigkeitsundurchlässige Membran (49), die an dem Zylinder angrenzend an das zweite Ende des Zylinders (12) befestigt ist, wobei die zweite gasdurchlässige, flüssigkeitsundurchlässige Membran (49) durch eine Probensonde (72) durchstechbar ist, so dass die Probensonde (72) durch die zweite gasdurchlässige, flüssigkeitsundurchlässige Membran zur Auslassseite (46) der Innenkammer (26) geführt werden kann,
wobei das Filterelement (16) ferner den Körper (50) mit einem ersten Ende (52), einem zweiten Ende (54) und einer Seitenwand (56), die sich vom ersten Ende zum zweiten Ende erstreckt, umfasst, wobei die Seitenwand (22) des Körpers (50) einen Durchgang (58) definiert, der sich vom ersten Ende zum zweiten Ende durch den Körper (50) erstreckt, und wobei sich die gasdurchlässige, flüssigkeitsundurchlässige Membran (48) über den gesamten Durchgang (58) erstreckt und wobei die Seitenwand (56) des Körpers (50) mindestens zwei ringförmige Vorsprünge (60) aufweist, die sich in gleitbarem Kontakt mit der Innenfläche des Zylinders (12) radial nach außen erstrecken, und wobei mindestens einer der Vorsprünge (60) mit der Innenfläche des Zylinders (12) in dichtendem Kontakt ist, wenn sich das Filterelement (16) in der ersten Position befindet, und wobei das Filterelement (16) mit dem Zylinder (12) zusammenwirkt, um mindestens einen Flüssigkeitsdurchgang (47) zwischen dem Filterelement (16) und dem Zylinder vorzusehen,
wobei der Flüssigkeitsdurchgang (47) durch einen Körper (50) und mehrere Nuten (27), die sich entlang zumindest eines Abschnitts der Innenfläche des Zylinders (12) erstrecken, definiert ist.

2. Vorrichtung zum Entfernen von Blasen aus einer Fluidprobe (32) mit einem Flüssigkeitsanteil und einem Gasanteil nach Anspruch 1 in Kombination mit einem Flüssigkeitsprobenanalysator mit der Probensonde (72), wobei das zweite Ende des Zylinders (12) mit dem Flüssigkeitsprobenanalysator verbunden ist.

3. Vorrichtung nach Anspruch 1, wobei sich die Nuten (27) in Umfangsrichtung um die Innenfläche des Zylinders (12) erstrecken oder
wobei die Nuten (27) parallel zueinander sind.

4. Vorrichtung nach Anspruch 1 oder 2, wobei der Zylinder (12) ein Anschlagelement aufweist, um in der zweiten Position mit dem Filterelement (16) in Eingriff zu kommen.

5. Vorrichtung nach Anspruch 1, wobei die gasdurchlässige, flüssigkeitsundurchlässige Membran (48) an dem Körper (50) angrenzend an das zweite Ende des Körpers (50) befestigt ist.

6. Vorrichtung nach Anspruch 1 oder 2, wobei die gasdurchlässige, flüssigkeitsundurchlässige Membran (48) aus einem Material gebildet ist, das Polytetraflyorethylen, Polypropylen und/oder Polyethylen umfasst.

7. Vorrichtung nach Anspruch 1, wobei der Körper (50) mehrere Durchgänge (58) aufweist, die sich vom ersten Ende zum zweiten Ende durch den Körper erstrecken, und wobei das Filterelement (16) ferner mehrere gasdurchlässige, flüssigkeitsundurchlässige Membranen aufweist, wobei sich mindestens eine der gasdurchlässigen, flüssigkeitsundurchlässigen Membranen über jeden der Durchgänge (58) des Körpers (50) erstreckt.

8. Vorrichtung nach Anspruch 7, wobei das Filterelement ferner mehrere poröse Filtermaterialien umfasst, die zwischen dem ersten Ende des Körpers (50) und jeder der gasdurchlässigen, flüssigkeitsundurchlässigen Membranen positioniert sind, um zu verhindern, dass feste Partikel mit den gasdurchlässigen, flüssigkeitsundurchlässigen Membranen in Kontakt kommen.

9. Vorrichtung nach Anspruch 7, wobei die mehreren gasdurchlässigen, flüssigkeitsundurchlässigen Membranen am Körper (50) angrenzend an das zweite Ende des Körpers (50) befestigt sind und/oder
wobei sich die Durchgänge (58) in einer parallelen Beziehung zueinander befinden und/oder
wobei die gasdurchlässige, flüssigkeitsundurchlässige Membran (48) aus einem Material gebildet ist, das Polytetraflyorethylen, Polypropylen und/oder Polyethylen umfasst.

10. Vorrichtung nach Anspruch 1 oder 2, wobei die zweite gasdurchlässige, flüssigkeitsundurchlässige Membran (49) aus einem Material gebildet ist, das Polytetraflyorethylen, Polypropylen und/oder Polyethylen umfasst.

11. Verfahren zum Entfernen von Blasen aus einer Fluidprobe (32) mit einem Flüssigkeitsanteil und einem Gasanteil, wobei das Verfahren Folgendes umfasst:
Empfangen eines Stroms der Fluidprobe (32) von einer Einlassöffnung (28) eines Zylinders mit einem ersten Ende, einem zweiten Ende, einer Seitenwand (22), die sich zwischen dem ersten Ende und dem zweiten Ende erstreckt, und einer Innenfläche, die eine Innenkammer definiert, wobei das erste Ende die Einlassöffnung (28) aufweist und das zweite Ende eine Auslassöffnung (30) aufweist;
Leiten der Fluidprobe (32) über die Einlassöffnung (28) in die Innenkammer (26);
Bewirken, dass die Fluidprobe (32) ein Filterelement berührt, das innerhalb der Innenkammer angeordnet ist, wobei das Filterelement eine Einlassseite (44) und eine Auslassseite (46) der Innenkammer definiert, wobei das Filterelement eine gasdurchlässige, flüssigkeitsundurchlässige Membran aufweist, wobei das Filterelement von einer ersten Position in eine zweite Position bewegbar ist;
Trennen zumindest eines Teils des Gasanteils der Flüssigkeitsprobe vom flüssigen Teil der Fluidprobe (32), indem bewirkt wird, dass die Fluidprobe die gasdurchlässige, flüssigkeitsundurchlässige Membran (48) des Filterelements in der ersten Position berührt, so dass zumindest ein Teil des Gasanteils der Fluidprobe (32) von der Einlassseite (44) zur Auslassseite (46) der Innenkammer (26) über das Filterelement verläuft, bevor bewirkt wird, dass das Filterelement in die zweite Position bewegt wird, so dass der flüssige Teil der Fluidprobe (32) von der Einlassseite (44) zur Auslassseite (46) gelangt; und
Sammeln zumindest eines Teils des flüssigen Teils der Fluidprobe (32) von der Auslassseite der Innenkammer, wobei zumindest ein Teil des flüssigen Teils der Fluidprobe (32) von der Auslassseite (46) der Innenkammer (26) durch Durchstechen einer zweiten gasdurchlässigen, flüssigkeitsundurchlässige Membran (49), die am Zylinder (12) angrenzend an das zweite Ende des Zylinders (12) befestigt ist, gesammelt wird, so dass eine Probensonde durch die zweite gasdurchlässige, flüssigkeitsundurchlässige Membran (49) zur Auslassseite geführt werden kann, um den flüssigen Teil der Fluidprobe (32) von der Auslassseite (46) der Innenkammer abzuziehen,
wobei das Filterelement (16) ferner den Körper (50) mit einem ersten Ende (52), einem zweiten Ende (54) und einer Seitenwand (56), die sich vom ersten Ende zum zweiten Ende erstreckt, umfasst, wobei die Seitenwand (22) des Körpers (50) einen Durchgang (58) definiert, der sich vom ersten Ende zum zweiten Ende durch den Körper (50) erstreckt, und wobei sich die gasdurchlässige, flüssigkeitsundurchlässige Membran (48) über den gesamten Durchgang (58) erstreckt und wobei die Seitenwand (56) des Körpers (50) mindestens zwei ringförmige Vorsprünge (60) aufweist, die sich in gleitbarem Kontakt mit der Innenfläche des Zylinders (12) radial nach außen erstrecken, und wobei mindestens einer der Vorsprünge (60) mit der Innenfläche des Zylinders (12) in dichtendem Kontakt ist, wenn sich das Filterelement (16) in der ersten Position befindet, und wobei das Filterelement (16) mit dem Zylinder (12) zusammenwirkt, um mindestens einen Flüssigkeitsdurchgang (47) zwischen dem Filterelement (16) und dem Zylinder vorzusehen,
wobei der Flüssigkeitsdurchgang (47) durch einen Körper (50) und mehrere Nuten (27), die sich entlang zumindest eines Abschnitts der Innenfläche des Zylinders (12) erstrecken, definiert ist.

12. Verfahren nach Anspruch 11, wobei das Filterelement ferner ein Filterelement umfasst, das zwischen der gasdurchlässigen, flüssigkeitsundurchlässigen Membran (48) und der Einlassseite (44) der Innenkammer angeordnet ist, wobei das mindestens eine Filterelement eine Einlassöffnung und eine Auslassöffnung aufweist, wobei die Einlassöffnung mit der Einlassseite (44) der Innenkammer (26) in Fluidverbindung steht, wobei die Auslassöffnung mit der gasdurchlässigen, flüssigkeitsundurchlässigen Membran in Fluidverbindung steht, um zumindest einen Teil der Fluidprobe (32) über das mindestens eine Filterelement und zu der gasdurchlässigen, flüssigkeitsundurchlässigen Membran (48) passieren zu lassen, wobei die gasdurchlässige, flüssigkeitsundurchlässige Membran vorzugsweise mehrere gasdurchlässige, flüssigkeitsundurchlässige Membranen (48) ist.

## Revendications

1. Appareil (10) pour éliminer des bulles d'un échantillon de fluide (32) ayant une partie liquide et une partie gazeuse, comprenant :
un cylindre (12) ayant une première extrémité (18), une seconde extrémité (20), une paroi latérale (22) s'étendant entre la première extrémité et la seconde extrémité, et une surface intérieure (24) définissant une chambre interne (26), la première extrémité ayant une ouverture d'entrée (28) et la seconde extrémité ayant une ouverture de sortie (30) ;
un élément filtrant (16) disposé à l'intérieur de la chambre interne (26) entre la première extrémité et la seconde extrémité de sorte que l'élément filtrant (16) définisse un côté entrée (44) et un côté sortie (46) de la chambre interne, l'élément filtrant (16) comportant au moins une première membrane (48) perméable aux gaz et imperméable aux liquides, l'élément filtrant (16) pouvant coulisser dans la chambre interne (26) du cylindre (12) d'une première position, permettant à la partie gazeuse de l'échantillon de fluide (32) de passer à travers l'au moins une membrane perméable aux gaz et imperméable aux liquides (48), à une seconde position permettant à la partie liquide de l'échantillon de fluide (32) de passer du côté entrée (44) au côté sortie (46) ; et
une seconde membrane perméable aux gaz et imperméable aux liquides (49) fixée au cylindre à proximité de la seconde extrémité du cylindre (12), la seconde membrane perméable aux gaz et imperméable aux liquides (49) pouvant être percée par une sonde d'échantillonnage (72) de sorte que la sonde d'échantillonnage (72) puisse passer à travers la seconde membrane perméable aux gaz et imperméable aux liquides jusqu'au côté sortie (46) de la chambre interne (26),
dans lequel l'élément filtrant (16) comprend en outre le corps (50) ayant une première extrémité (52), une seconde extrémité (54), et une paroi latérale (56) s'étendant de la première extrémité à la seconde extrémité, la paroi latérale (22) du corps (50) définissant un passage (58) s'étendant à travers le corps (50) de la première extrémité à la seconde extrémité, et dans lequel la membrane (48) perméable aux gaz et imperméable aux liquides s'étend transversalement à la totalité du passage (58), et dans lequel la paroi latérale (56) du corps (50) comporte au moins deux saillies annulaires (60) s'étendant radialement vers l'extérieur en contact de coulissement avec la surface intérieure du cylindre (12), et dans lequel au moins une des saillies (60) est en contact étanche avec la surface intérieure du cylindre (12) lorsque l'élément filtrant (16) est dans la première position, et dans lequel l'élément filtrant (16) coopère avec le cylindre (12) pour former au moins un passage de liquide (47) entre l'élément filtrant (16) et le cylindre,
dans lequel le passage de liquide (47) est défini par un corps (50) et une pluralité de rainures (27) s'étendant le long d'au moins une partie de la surface intérieure du cylindre (12).

2. Appareil pour enlever des bulles d'un échantillon de fluide (32) ayant une partie liquide et une partie gazeuse selon la revendication 1 en combinaison avec un analyseur d'échantillon de liquide ayant la sonde d'échantillonnage (72), dans lequel la seconde extrémité du cylindre (12) est reliée à l'analyseur d'échantillon de liquide.

3. Appareil selon la revendication 1, dans lequel les rainures (27) s'étendent circonférentiellement autour de la surface intérieure du cylindre (12), ou
dans lequel les rainures (27) sont parallèles entre elles.

4. Appareil selon la revendication 1 ou 2, dans lequel le cylindre (12) comporte un élément d'arrêt destiné à entrer en prise avec l'élément filtrant (16) dans la seconde position.

5. Appareil selon la revendication 1, dans lequel la membrane perméable aux gaz et imperméable aux liquides (48) est fixée au corps (50) à proximité de la seconde extrémité du corps (50).

6. Appareil selon la revendication 1 ou 2, dans lequel la membrane perméable aux gaz et imperméable aux liquides (48) est formée à partir d'un matériau comprenant au moins l'un du polytétrafluoréthylène, du polypropylène et du polyéthylène.

7. Appareil selon la revendication 1, dans lequel le corps (50) a une pluralité de passages (58) s'étendant à travers le corps de la première extrémité à la seconde extrémité, et dans lequel l'élément filtrant (16) a en outre une pluralité de membranes perméables aux gaz et imperméables aux liquides, au moins une des membranes perméables aux gaz et imperméables aux liquides s'étendant transversalement à chacun des passages (58) du corps (50).

8. Appareil selon la revendication 7, dans lequel l'élément filtrant comprend en outre une pluralité de matériaux filtrants poreux positionnés entre la première extrémité du corps (50) et chacune des membranes perméables aux gaz et imperméables aux liquides pour empêcher les particules solides d'entrer en contact avec les membranes perméables aux gaz et imperméables aux liquides.

9. Appareil selon la revendication 7, dans lequel la pluralité de membranes perméables aux gaz et imperméables aux liquides sont fixées au corps (50) à proximité de la seconde extrémité du corps (50), et/ou
dans lequel les passages (58) sont parallèles entre eux, et/ou dans lequel la membrane perméable aux gaz et imperméable aux liquides (48) est formée à partir d'un matériau comprenant au moins l'un du polytétrafluoréthylène, du polypropylène et du polyéthylène.

10. Appareil selon la revendication 1 ou 2, dans lequel la seconde membrane perméable aux gaz et imperméable aux liquides (49) est formée à partir d'un matériau comprenant au moins l'un du polytétrafluoréthylène, du polypropylène et du polyéthylène.

11. Procédé d'élimination de bulles d'un échantillon de fluide (32) ayant une partie liquide et une partie gazeuse, le procédé comprenant :
la réception d'un flux de l'échantillon de fluide (32) depuis une ouverture d'entrée (28) d'un cylindre ayant une première extrémité, une seconde extrémité, une paroi latérale (22) s'étendant entre la première extrémité et la seconde extrémité, et une surface intérieure définissant une chambre interne, la première extrémité ayant l'ouverture d'entrée (28) et la seconde extrémité ayant une ouverture de sortie (30) ;
l'introduction de l'échantillon de fluide (32) dans la chambre interne (26) par l'ouverture d'entrée (28) ;
la mise en contact de l'échantillon de fluide (32) avec un élément filtrant disposé à l'intérieur de la chambre interne, l'élément filtrant définissant un côté entrée (44) et un côté sortie (46) de la chambre interne, l'élément filtrant comportant une membrane perméable aux gaz et imperméable aux liquides, l'élément filtrant étant déplaçable d'une première position à une seconde position ;
la séparation d'au moins une partie de la partie gazeuse de l'échantillon de liquide de la partie liquide de l'échantillon de fluide (32) en mettant l'échantillon de fluide en contact avec la membrane perméable aux gaz et imperméable aux liquides (48) de l'élément filtrant dans la première position de sorte qu'au moins une partie de la partie gazeuse de l'échantillon de fluide (32) passe à travers l'élément filtrant du côté entrée (44) au côté sortie (46) de la chambre interne (26) avant de déplacer l'élément filtrant jusqu'à la seconde position de sorte que la partie liquide de l'échantillon de fluide (32) passe du côté entrée (44) au côté sortie (46) ; et
la collecte d'au moins une partie de la partie liquide de l'échantillon de fluide (32) depuis le côté sortie de la chambre interne, au moins une partie de la partie liquide de l'échantillon de fluide (32) étant collectée depuis le côté sortie (46) de la chambre interne (26) en perçant une seconde membrane perméable aux gaz et imperméable aux liquides (49) fixée au cylindre (12) à proximité de la seconde extrémité du cylindre (12) de sorte qu'une sonde d'échantillonnage puisse être introduite à travers la seconde membrane perméable aux gaz et imperméable aux liquides (49) jusqu'au côté sortie pour extraire la partie liquide de l'échantillon de fluide (32) depuis le côté sortie (46) de la chambre interne,
dans lequel l'élément filtrant (16) comprend en outre le corps (50) ayant une première extrémité (52), une seconde extrémité (54), et une paroi latérale (56) s'étendant de la première extrémité à la seconde extrémité, la paroi latérale (22) du corps (50) définissant un passage (58) s'étendant à travers le corps (50) de la première extrémité à la seconde extrémité, et dans lequel la membrane (48) perméable aux gaz et imperméable aux liquides s'étend transversalement à la totalité du passage (58), et dans lequel la paroi latérale (56) du corps (50) comporte au moins deux saillies annulaires (60) s'étendant radialement vers l'extérieur en contact de coulissement avec la surface intérieure du cylindre (12), et dans lequel au moins une des saillies (60) est en contact étanche avec la surface intérieure du cylindre (12) lorsque l'élément filtrant (16) est dans la première position, et dans lequel l'élément filtrant (16) coopère avec le cylindre (12) pour former au moins un passage de liquide (47) entre l'élément filtrant (16) et le cylindre,
dans lequel le passage de liquide (47) est défini par un corps (50) et une pluralité de rainures (27) s'étendant le long d'au moins une partie de la surface intérieure du cylindre (12).

12. Procédé selon la revendication 11, dans lequel l'élément filtrant comprend en outre un composant filtrant interposé entre la membrane perméable aux gaz et imperméable aux liquides (48) et le côté entrée (44) de la chambre interne, l'au moins un composant filtrant ayant un orifice d'entrée et un orifice de sortie, l'orifice d'entrée étant en communication fluidique avec le côté entrée (44) de la chambre interne (26), l'orifice de sortie étant en communication fluidique avec la membrane perméable aux gaz et imperméable aux liquides pour permettre à au moins une partie de l'échantillon de fluide (32) de passer à travers l'au moins un composant filtrant et vers la membrane perméable aux gaz et imperméable aux liquides (48), dans lequel la membrane perméable aux gaz et imperméable aux liquides est, de préférence, une pluralité de membranes perméables aux gaz et imperméables aux liquides (48).
